Europäisches Patentamt

(19) European Patent Office

Office européen des brevets

(11) Numéro de publication: **0 132 421**
**B1**

(12) **FASCICULE DE BREVET EUROPÉEN**

(45) Date de publication du fascicule du brevet:
07.02.90

(21) Numéro de dépôt: 84401221.1

(22) Date de dépôt: 14.06.84

(51) Int. Cl. ⁵: **G 03 B 42/02**

(54) Appareil d'examen radiologique et radiographique.

(30) Priorité: 24.06.83 FR 8310502

(43) Date de publication de la demande:
30.01.85 Bulletin 85/05

(45) Mention de la délivrance du brevet:
07.02.90 Bulletin 90/06

(84) Etats contractants désignés:
DE GB NL

(56) Documents cité:
DE-A-2 744 139
DE-A-3 022 248
DE-A-3 036 932
FR-A-2 161 229
FR-A-2 267 078
US-A-3 222 518

(73) Titulaire: GENERAL ELECTRIC CGR S.A.
100, rue Camille-Desmoulins
F-92130 Issy les Moulineaux (FR)

(72) Inventeur: Caugant, Jean
THOMSON-CSF SCPI 173, bld Haussmann
F-75379 Paris Cedex 08 (FR)
Inventeur: Stephan, Guy
THOMSON-CSF SCPI 173, bld Haussmann
F-75379 Paris Cedex 08 (FR)

(74) Mandataire: Ballot, Paul Denis Jacques
Cabinet Ballot 7, rue le Sueur
F-75116 Paris (FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBERGRAF, STOCKHOLM 1990

## Description

L'invention se rapporte à un appareil d'examen radiologique et radiographique. Elle s'applique notamment dans le domaine médical où il est fait un grand usage de tels appareils.

Un appareil d'examen radiologique comporte essentiellement une source radioactive, et un détecteur de rayonnements situé dans le champ scopique de cette source. Un patient à examiner est allongé sur un plateau porte-patient pour pouvoir être mis en place entre la source radioactive et le détecteur sur le chemin du rayonnement. Le détecteur est relié à un dispositif de visualisation qui donne une image radiologique d'une zone du patient irradiée par la source radioactive. Un appareil d'examen radiographique comporte également une source radioactive, un plateau porte-patient et en lieu et place du détecteur un film radio-sensible pour donner une image radiographique de la zone du patient irradiée.

Pour des raisons de sécurité de manipulation, les films radiosensibles sont contenus dans des cassettes en général normalisées, adaptées aux dimensions des films. Une cassette contenant un film radio-sensible est mise en place dans le champ scopique de la source avant irradiation au moyen d'un porte-cassette. Pour obtenir une meilleure définition de l'image radiographique, les cassettes et donc le porte-cassette sont disposés le plus près possible du plateau porte-patient. Il est connu d'accrocher le porte-cassette au plateau lui-même. De manière à permettre la prise d'image radiographique de l'une quelconque des parties du corps du patient, la fixation du porte-cassette au plateau s'effectue au moyen de glissières réalisées dans ce plateau et assurant, pour une partie quelconque du patient, une mise en place optimale de la cassette.

Un détecteur de rayonnement radioactif comporte un amplificateur de luminance. Les amplificateurs de luminance ont pour objet essentiel d'amplifier le rayonnement irradiant, ayant traversé le patient, en le transformant en un rayonnement radioélectrique plus intense et sur lequel on effectue ensuite la détection en général au moyen d'une caméra vidéo. Pour des raisons identiques à celle de la radiographie, l'amplificateur de luminance de la chaîne radiologique doit être placé le plus près possible du plateau porte-patient.

Cette contrainte de proximité dans les deux cas empêche que le porte-cassette et que l'amplificateur de luminance puissent se trouver en permanence ensemble à l'aplomb de la source radioactive pour permettre des usages successifs, et alternativement selon chacun des deux modes, de l'appareil mixte d'examen radiologique et radiographique. Cette solution est d'autant moins praticable que le porte-cassette comporte une embase métallique, pour maintenir la cassette sous toute sa surface, et que cette embase métallique constitue un obstacle pour le rayonnement à mesurer dans le cas d'une utilisation radiologique. Pour remédier à cet inconvénient, il est connu, lors d'un usage radiologique, de déplacer le porte-cassette parallèlement au plan du plateau pour qu'il sorte du champ scopique de la source. En usage radiographique on éloigne l'amplificateur de luminance pour permettre la mise en place du porte-cassette. Une telle réalisation est par exemple présentée dans un brevet américain 3 222 518 publié le 7 Décembre 1965.

Dans l'utilisation courante, un tel appareil d'examen mixte fonctionne alternativement en radiologie pour repérer les zones du corps du patient à examiner et en radiographie pour prendre une image radiographique de la zone repérée. Hormis les problèmes de centrage du porte-cassette sur la zone étudiée en radiologie, les dispositifs de l'art antérieur présentent deux inconvénients. Le premier inconvénient se situe dans la nécessité des manipulations iterratives du porte-cassette et de l'amplificateur de luminance au cours d'une série d'examen. Le deuxième inconvénient est un problème d'encombrement. En effet lorsqu'il est déplacé hors du champ scopique de la source, le porte-cassette constitue une gêne pour le déplacement relatif du plateau porte-patient par rapport à l'alignement source radioactive-amplificateur de luminance. Si on appelle position de garage la position du porte-cassette sous le plateau porte-patient lors d'une application de radiologie, cette position de garage doit se situer au-delà du corps du patient soit au-delà des pieds soit au-delà de la tête. Ceci permet une investigation générale par déplacement du champ scopique tout du long du corps du patient. Ce surcroît de place est encombrant. Dans d'autres solutions, où l'on n'utilise pas un plateau porte-patient plus grand que nécessaire, on est amené à déterminer deux positions garage: une vers les pieds et une vers la tête. En cas d'erreur de choix de la position garage retenue, il y a lieu d'effectuer une manipulation supplémentaire pour transférer le porte-cassette dans la position garage où il ne le gêne pas. Ces manipulations supplémentaires font perdre du temps et contrarient des opérateurs utilisant l'appareil d'examen.

L'invention a pour objet de remédier aux inconvénients cités, en proposant un appareil d'examen radiologique et radiographique comportant un porte-cassette ne coulissant plus longitudinalement par rapport au plateau porte-patient mais pouvant, en position garage, venir coulisser autour d'un fût contenant l'amplificateur de luminance.

L'invention est définie dans la revendication 1.

La présente invention sera mieux comprise à la lecture de la description qui suit et à l'examen des figures qui l'accompagnent. Cette description n'est pas limitative des caractéristiques de l'invention. Sur ces figures les mêmes repères désignent les mêmes éléments. Ces figures représentent:

- figure 1: une vue en perspective du porte-cassette inclus dans l'appareil de l'invention;
- figure 2: une vue d'une réalisation particulière d'un porte-cassette mis en oeuvre dans l'appareil de l'invention;

- figure 3: une vue du mode d'insertion d'une cassette dans le porte-cassette de la figure 2.
- figure 4 une vue d'une cassette utilisée;
- figure 5: une vue d'une partie du porte-cassette.

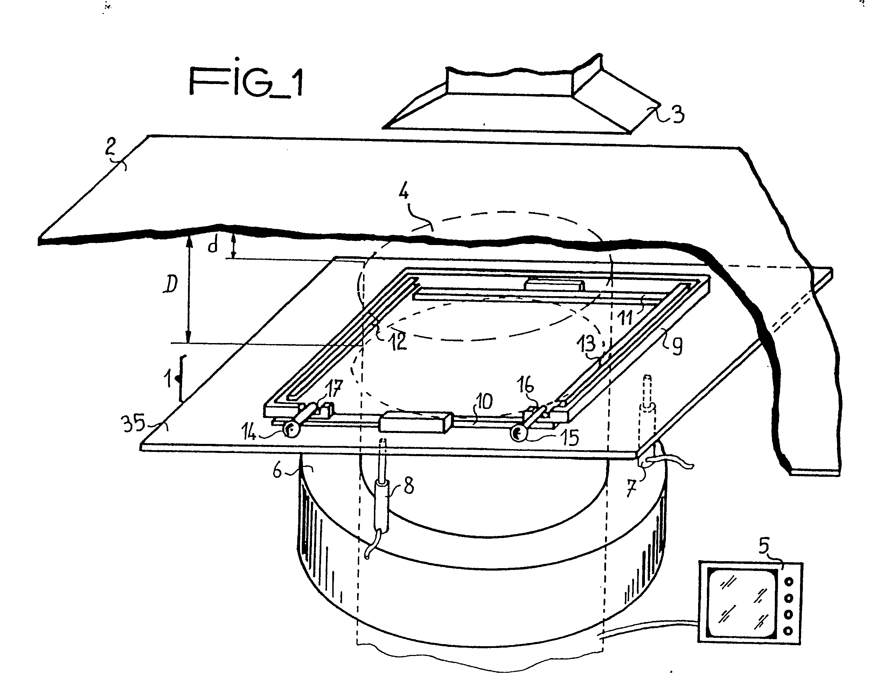

La figure 1 représente un porte-cassette escamotable 1 adapté à un appareil d'examen radiologique et radiographique selon l'invention comportant un plateau, porte-patient 2, une source radioactive 3 et un fût cylindrique 4, dessiné en tirets, et contenant un amplificateur de luminance non figuré. Cet amplificateur est relié à un moyen de visualisation 5, qui élabore une image radiologique de la zone du patient irradiée par la source 3. Dans l'utilisation radiologique qui est représentée, la face supérieure de l'amplificateur de luminance est placée à une distance d de la face inférieure du plateau 2. Le porte-cassette 1 est escamotable par coulissement autour du fût 4 pour que son plan se trouve à une distance D du plateau 2. La distance D est supérieure à d. Le porte-cassette 1 constitue donc un équipage mobile emporté par des moyens de transfert quelconques, et plus particulièrement ici, par un jeu de trois vérins disposés régulièrement autour du fût 4 et prenant appui d'une part sur un bâti 6 solidaire du bâti supportant le fût 4. Des trois vérins seuls deux, les vérins 7 et 8, ont été représentés. Ces vérins prennent appui d'autre part sous le porte-cassette 1. Le porte-cassette 1 comporte des moyens 9 pour réaliser un évidement de dimensions variables en son milieu. Dans l'exemple décrit, en position garage, cet évidement est déterminé par deux jeux de mors, le jeu des mors 10 et 11 et le jeu des mors 12 et 13.

Cet évidement est ainsi légèrement plus grand que la section du fût 4 rappelé par une courbe en pointillée. Chaque jeu de mors est manoeuvrable manuellement au moyen d'une poignée respectivement, la poignée 14 pour le jeu 10 et 11, la poignée 15 pour le jeu 12 et 13. On remarque sur la figure 1, que les moyens 9 comportent des encoches respectivement 17 et 16 pour assurer le blocage des poignées 14 et 15 dans une position telle que les mors 10 et 11 ou 12 et 13 ne viennent pas prendre appui contre le fût 4.

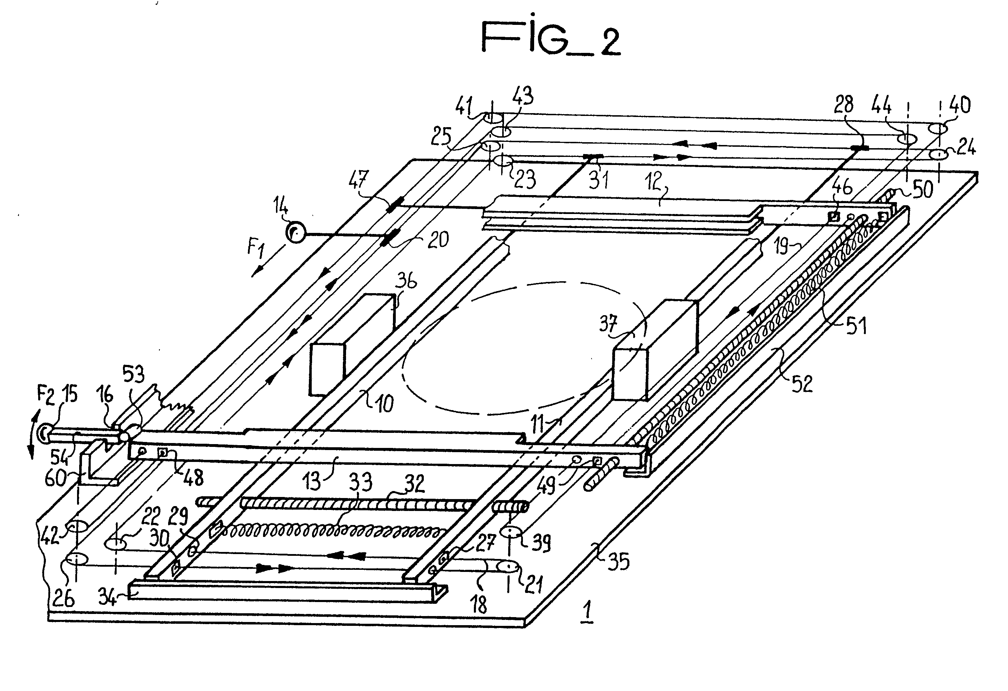

La manipulation des mors 10 à 13 au moyen des poignées 14 et 15 est représentée sur la figure 2. Ces moyens ne sont qu'un exemple pour réaliser l'évidement. Ils pourraient avoir tout autre conformation tendant à produire un même résultat. Ils comprennent les jeux de mors 10 et 11, et 12 et 13. Chacun de ces jeux de mors est entraîné par un câble sans fin. Le jeu de mors 10 et 11 est entraîné par le câble 18 rappelé par des doubles flèches, et le jeu de mors 12 et 13 est entraîné par le câble 19 rappelé par des flèches uniques. Par une action exercée sur la poignée 14 dans le sens de la flèche $F_1$ tendant à rapprocher cette poignée 14 du milieu du porte-cassette 1, la poignée 14, solidaire du câble 18 par la fixation 20, entraîne le câble 18 dans le sens indiqué par les double flèches. Le câble 18 circule sur le pourtour du porte-cassette de telle manière qu'en tous endroits il présente deux portions allant en sens inverse l'une de l'autre. Le câble 18 est tendu entre des poulies folles 21 à 26. La portion du câble 18 situé entre la poulie 21 et la poulie 22 est rendue solidaire par une fixation 27 d'une extrémité du mors 11 tandis que la portion de ce câble située entre la poulie 24 et la poulie 25 est rendue solidaire par la fixation 28 de l'autre extrémité de ce mors. Par une disposition judicieuse, lorsque le câble 18 circule sur ces poulies, ces deux portions sont entraînées dans un sens identique amenant le mors 11 à se déplacer parallèlement à lui-même.

Les poulies 21 à 26 se trouvent dans un même plan. On remarque que le câble 18 passe librement dans un trou 29 effectué dans une extrémité du mors 10. Ce mors 10 est entraîné par le câble 18 au moyen de fixations 30 et 31 situés respectivement sur les portions du câble 18 contenues entre les poulies 21 et 26 et 23 et 24. Les poulies 21 à 24 sont disposées au sommet d'un quadrilatère sensiblement carré. Les poulies 25 et 26 se trouvent respectivement dans le prolongement des diagonales 21 - 23 et 24 - 22 de ce quadrilatère. Le câble 18 ne subissant pas d'élongation lors de son mouvement, on constate que le mors 10 se déplace parallèlement à lui-même en un sens opposé de celui du mors 11, et, que pour un déplacement donné du câble 18, les grandeurs de déplacement de chacun de ces mors sont identiques.

Pour permettre un parfait parallèlisme du déplacement des mors 10 et 11, un jeu de barres coulissantes dont seule la barre 32 est représentée assure le maintien relatif des mors 10 et 11. Par ailleurs un moyen de rappel, symbolisé par un ressort 33 travaillant en extension, est fixé par ses extrémités à chacun des mors 10 et 11. Il tend ainsi à rapprocher les mors 10 et 11 l'un de l'autre. Les mors 10 et 11 sont enfin supportés par un jeu de cornières, telles que 34, solidaires du bâti 35 du porte-cassette 1, et prenant appui sous les extrémités de ces mors. Les axes des poulies 21 et 26 sont également fixés au support 35. On n'attachera pas d'importance au bras de levier reliant la poignée 14 à sa fixation 20 au câble 18: celui-ci ne figure que pour aérer le dessin. Dans la pratique la poignée 14 est fixée directement sur le câble 18. Les mors 10 et 11 entraînent deux butées respectivement 36 et 37 qui émergent du plan formé par les mors 10 et 11.

Situé dans un plan supérieur au plan des mors 10 et 11 se trouve le plan des mors 12 et 13. Ce dernier plan coupe les faces actives des butées 36 et 37 qui seront étudiées ultérieurement. Les mors 12 et 13 sont entraînés, comme les mors 10 et 11, par un câble 19 circulant entre les poulies 39 à 44. Les poulies 39 à 43 sont des poulies folles ayant un axe commun respectivement avec les poulies 21, 24, 25, 26 et 23. La poulie folle 44 se trouve près de la poulie 40 dans la diagonale des poulies 40, 42. Les mors 12 et 13 sont entraînés, comme les mors 10 et 11, par des fixations respectivement 46 et 47 et 48 et 49 au câble 19. Les mors 12 et 13 comportent également une barre 50 pour maintenir leur parallèlisme, un ressort 51 pour assurer leur rappel et un jeu de glissières telles que 52 et 60 pour les supporter dans leur déplacement. Les mors 12 et 13 ont un profil utile en forme de lettre C pour prévenir les déplacements tant latéraux que verticaux d'une cassette. Ainsi, lorsqu'une cassette est introduite entre les mors 12 et 13, elle est maintenue dans deux directions par ces mors, et dans la troisième direction par les faces en vis à vis des butées 36 et 37.

La poignée 15, assurant comme la poignée 14 la mise en mouvement d'un câble, est ici fixée dans le prolongement du mors 13 par une charnière 53. Cette charnière 53 permet à la poignée 15 de pivoter dans le sens de la double flèche $F_2$ de telle manière qu'en position de garage le manche 54 de cette poignée 15 vienne s'incruster dans l'encoche 16 de la glissière 60. Ce faisant l'action du ressort de rappel 51 est neutralisée, et les mors 12 et 13 sont maintenus dans une position garage. Cette disposition présente un avantage qui est celui de disposer la poignée 15 du même côté que la poignée 14 afin de mettre l'ensemble de ces deux poignées 14 et 15 en face de l'opérateur utilisant l'appareil mixte.

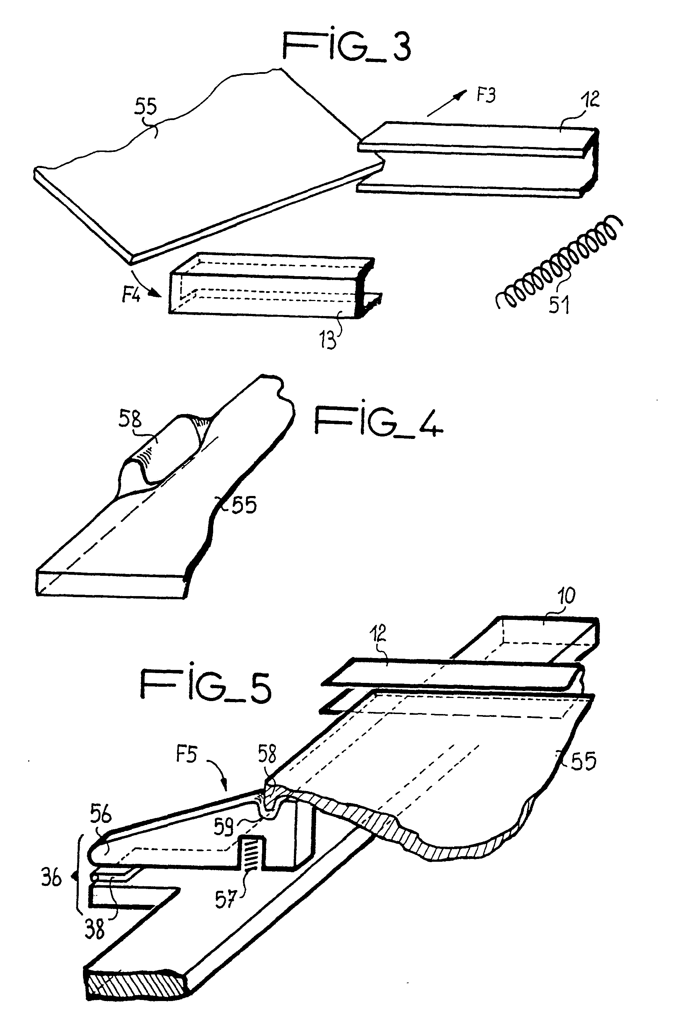

Les figures 3 à 5 représentent la phase d'introduction d'une cassette 55 dans le porte-cassette. Dans un premier temps l'opérateur déverrouille la poignée 15 et laisse les mors 12 et 13 venir se rapprocher sous l'effet du ressort de rappel 51. Puis saisissant la cassette 55, l'opérateur en introduit un angle dans la rainure constituée par le mors 12. En exerçant sur la cassette 55 une pression dans le sens de la flèche $F_3$ il écarte simultanément les deux mors 12 et 13 l'un de l'autre. Lorsque l'écartement de ceux-ci est suffisant, il redresse (flèche $F_4$) la cassette 55 pour l'introduire correctement dans les mors 12 et 13.

La butée 36 (figure 5) est effaçable. Dans un exemple particulier de réalisation, elle est en forme générale de dièdre et est articulée à son sommet 56 par une charnière 38 sur le mors 10. Elle peut s'effacer dans le sens de la flèche $F_5$ dans le sens inverse d'un ressort de rappel 57 agissant en compression. Ce ressort de rappel 57 prend appui d'une part sur la butée 36 et d'autre part sur le mors 10. Les cassettes 55 (figure 4) comportent généralement une protubérance 58 située sur leur partie inférieure: la cassette 55 de la figure 4 est représentée à l'envers. La protubérance 58 vient s'engager, lors de la mise en place de la cassette, dans une encoche 59 réalisée dans le plan supérieur du dièdre de la butée 36. Une fois ce résultat atteint l'opérateur dévérouille la poignée 14 maintenue dans l'encoche 17, et il conduit cette poignée pour déplacer le mors 10 en direction du mors 11 de telle manière que l'extrémité avant de la cassette 55 vienne en butée sur face active de la butée 37. Les fixations des mors sur leurs câbles respectifs sont ajustées expérimentalement pour que ces mors se trouvent en permanence à égale distance du milieu de l'évidement constitué. Ce faisant, quelque soit la dimension de la cassette, même s'il elle n'est pas normalisée, le centrage sur la zone radiographiée est obtenu automatiquement.

Dans une version tout automatique, l'entraînement des mors peut se faire par des moteurs actionnables par des boutons de commande. Enfin, la mise en action des moyens de coulissement 7 et 8 peut être exercée en même temps que la mise en action de moyens tendant à éloigner le fût 4 du panneau porte-patient. Cependant, compte tenu du faible encombrement en hauteur du porte-cassette de l'invention en particulier parce qu'il ne comporte plus d'embase métallique, l'éloignement du fût 4 devient facultatif.

## Revendications

1. Appareil d'examen radiologique et radiographique comportant un plateau porte-patient (2) et une source radioactive (3) située en regard d'une première face du plateau pour soumettre une zone du patient à une irradiation, un amplificateur de luminance comportant un fût (4) situé en regard de l'autre face du plateau à l'aplomb de la source radioactive pour élaborer une image radiologique de la zone irradiée, et un porte-cassette (1) escamotable constituant un équipage mobile (35) susceptible de recevoir une cassette radiosensible (55) et de venir occuper une position de travail entre la deuxième face du plateau et le fût de l'amplificateur de luminance pour permettre d'élaborer une image radiographique de la zone irradiée, caractérisé en ce que le porte-cassette est mobile perpendiculairment au plan du plateau entre sa position de travail et une position de garage dans laquelle il est davantage éloigné de la deuxième face du plateau que dans sa position de travail et en ce qu'il comporte des moyens (10 - 15) permettant de mettre en place ou de retirer la cassette radiosensible lorsque le porte cassette se trouve en position de travail, ces moyens définissant, en l'absence de la cassette, un évidement de dimension variable en son milieu pour recevoir, en position garage du porte cassette, le fût de l'amplificateur de luminance.

2. Appareil selon la revendication 2, caractérisé en ce que les moyens de réalisation de l'évidement comportent deux jeux (10 -11 et 12 - 13) de deux mors en vis à vis, chacun des mors d'un jeu pouvant se mouvoir dans une direction ($F_1$) perpendiculaire à la direction d'alignement de la source radioactive sur l'amplificateur de luminance, les directions de mouvement de chacun des deux jeux étant sensiblement perpendiculaires entre elles.

3. Appareil selon la revendication 1, caractérisé en ce qu'il comporte des moyens (27, 28, 30, 31, 46 - 49) pour que le milieu de l'évidement soit situé à l'aplomb du centre de l'amplificateur de luminance.

4. Appareil selon la revendication 3, caractérisé en ce que les moyens de réalisation de l'évidement comportent des moyens pour déplacer symétriquement (18, 19) les deux mors d'un jeu par rapport au milieu du porte-cassette.

5. Appareil selon la revendication 4, caractérisé en ce que les moyens de déplacement symétrique d'un jeu de mors comportent un câble (18) sans fin entraînant les deux mors, empruntant un chemin entourant partiellement (21 à 26) le porte-cassette, de telle manière qu'en chaque endroit de ce chemin existent deux portions de ce câble sensiblement parallèles et circulant dans des directions inverses l'une de l'autre.

6. Appareil selon la revendication 5, caractérisé en ce que les moyens de déplacement symétrique d'un jeu de mors comportent un moyen (33) de rappel élastique tendant à rapprocher l'un de l'autre les deux mors en vis à vis.

7. Appareil selon la revendication 5, caractérisé en ce que le câble d'un moyen de déplacement symétrique peut être mis en mouvement manuellement par une poignée (14).

8. Appareil selon la revendication 4, caractérisé en ce que les moyens de déplacement symétrique d'un jeu de mors comporte une position (17) autoblocante dite garage dans laquelle l'évidement est à une dimension suffisante pour permettre le coulissement de l'amplificateur de luminance.

9. Appareil selon la revendication 5, caractérisé en ce que les deux jeux de mors sont situés dans des plans superposés.


**Patentansprüche**

1. Radiologisches und radiographisches Untersuchungsgerät mit einer Patienten-Tragplatte (2), einer radioaktiven Quelle (3), die einer ersten Plattenseite gegenüberliegt, um eine Körperzone des Patienten einer Bestrahlung auszusetzen, ein Leuchtdichteverstärker mit einer Säule (4), die der anderen Plattenseite lotrecht von der radioaktiven Quelle gegenüberliegt, um ein radiologisches Bild der bestrahlten Körperzone zu erzeugen, und einen versenkbaren Kassettenträger (1), der eine bewegliche Ausrüstung (35) bildet, die dazu geeignet ist, eine strahlenempfindliche Kassette (55) aufzunehmen und eine Arbeitsstellung zwischen der zweiten Plattenseite und der Leuchtdichteverstärker-Säule einzunehmen, um die Erzeugung eines radiographischen Bildes der bestrahlten Körperzone zu ermöglichen, dadurch gekennzeichnet, daß der Kassettenträger senkrecht zur Plattenebene zwischen seiner Arbeitsstellung und einer Ausweichstellung beweglich ist, in der er von der zweiten Plattenseite weiter entfernt ist als in seiner Arbeitsstellung, und dadurch, daß er Mittel (10 - 15) umfaßt, die ermöglichen, die strahlungsempfindliche Kassette einzulegen oder herauszunehmen, wenn der Kassettenträger sich in Arbeitsstellung befindet, wobei diese Mittel bei Fehlen der Kassette eine Aussparung veränderlicher Abmessung in seiner Mitte definieren, um in Ausweichstellung des Kassettenträgers die Leuchtdichteverstärker-Säule aufzunehmen.

2. Gerät nach Anspruch 2, dadurch gekennzeichnet, daß die Mittel zur Durchführung der Aussparung zwei Sätze (10 - 11 und 12 - 13) von zwei gegenüberliegenden Spannbacken umfassen, wobei sich jede Spannbacke eines Satzes in eine Richtung (F₁) bewegen kann, die senkrecht zur Zentrierrichtung der radioaktiven Quelle auf dem Leuchtdichteverstärker ist, wobei die Bewegungsrichtungen jedes der beiden Sätze untereinander etwa senkrecht sind.

3. Gerät nach Anspruch 1, dadurch gekennzeichnet, daß es Mittel (27, 28, 30, 31, 46 - 49) umfaßt, damit die Mitte der Aussparung lotrecht vom Zentrum des Leuchtdichteverstärkers gelegen ist.

4. Gerät nach Anspruch 3, dadurch gekennzeichnet, daß die Mittel zur Durchführung der Aussparung Mittel umfassen, um die beiden Spannbacken eines Satzes in Bezug auf die Mitte des Kassettenträgers symmetrisch (18, 19) zu verschieben.

5. Gerät nach Anspruch 4, dadurch gekennzeichnet, daß die Mittel der symmetrischen Verschiebung eines Spannbackensatzes ein Endloskabel (18) umfassen, das die beiden Spannbacken mitzieht, wobei es einem Weg folgt, der teilweise (21 bis 26) den Kassettenträger umläuft, so daß an jeder Stelle dieses Weges zwei etwa parallele Abschnitte dieses Kabels vorkommen, die in einander entgegengesetzten Richtungen verlaufen.

6. Gerät nach Anspruch 5, dadurch gekennzeichnet, daß die Mittel der symmetrischen Verschiebung eines Spannbackensatzes ein Mittel (33) für die elastische Rückholung umfassen, das dazu neigt, die beiden gegenüberliegenden Spannbacken einander anzunähern.

7. Gerät nach Anspruch 5, dadurch gekennzeichnet, daß das Kabel eines symmetrischen Verschiebungsmittels manuell mittels eines Griffs (14) bewegt werden kann.

8. Gerät nach Anspruch 4, dadurch gekennzeichnet, daß die Mittel der symmetrischen Verschiebung eines Spannbackensatzes eine selbstblockierende sogenannte Ausweichstellung (17) umfaßt, in der die Aussparung eine ausreichende Abmessung hat, um das Verschieben des Leuchtdichteverstärkers zu ermöglichen.

9. Gerät nach Anspruch 5, dadurch gekennzeichnet, daß sich die beiden Spannbackensätze auf übereinanderliegenden Ebenen befinden.

**Claims**

1. A radiological and radiographic examining apparatus comprising a patient support stage (2), a radioactive source (3) situated opposite to a first face of the stage in order to submit a zone of the patient to irradiation, a luminance amplifier with a pedestal (4) situated opposite the other face of the stage vertically in line with the radioactive source in order to process a radiological image of the irradiated zone, and a retractable cassette carrier (1) constituting a mobile fitting (35) adapted to receive a radiosensitive cassette (55) and to assume a working position between the second face of the stage and the pedestal of the luminance amplifier in order to make possible the processing of a radiographic image of the irradiated zone, characterized in that the cassette carrier is able to move perpendicularly to the plane of the stage between its working position and a standby position in which it is further removed from the second face of the stage than in its working position and in that it comprises means (10 - 15) making it possible to put into position or to retract the radiosensitive cassette when the cassette carrier is in its working position, said means defining, absent the cassette, an opening of variable dimension in its middle in order to receive, in the standby position of the cassette carrier, the pedestal of luminance amplifier.

2. The apparatus as claimed in claim 2, characterized in that the means for constituting the opening comprise two sets (10 - 11 and 12 - 13) of two jaws opposite to each other, each of the jaws of a set being able to move in a direction ($F_1$) perpendicular to the direction of alignment of the radioactive source with respect to the luminance amplifier, the directions of movement of each of two jaws being essentially perpendicular to each other.

3. The apparatus as claimed in claim 1, characterized in that it comprises means (27, 28, 30, 31 and 46 - 49) in order to ensure that the middle of the opening is situated in vertical alignment with the middle of the luminance amplifier.

4 The apparatus as claimed in claim 3, characterized in that the means forming the opening comprise means for symmetrically displacing (18 and 19) the two jaws of one set in relation to the middle of the cassette carrier.

5. The apparatus as claimed in claim 4, characterized in that the means for causing symmetrical displacement of one set of jaws comprise an endless cable (18) driving the two jaws complying with a path partially extending around (21 through 26) the cassette carrier in such a manner that at each point of this path there are two portions of this cable essential parallel and moving in directions opposite to each other.

6. The apparatus as claimed in claim 5, characterized in that the means for causing symmetrical displacement of one set of jaws comprise an elastic returning means (33) tending to move the two jaws together in a manner opposite to each other.

7. The apparatus as claimed in claim 5, characterized in that the cable of one means for causing symmetrical displacement may be manually moved by a handle (14).

8. The apparatus as claimed in claim 4, characterized in that the means for causing symmetrical displacement of a set of jaws has a self-locking position (17) termed the standby position in which the opening is set to a sufficient size to permit the sliding of the luminance amplifier.

9. The apparatus as claimed in claim 5, characterized in that the two sets of jaws are situated in superposed planes.

6

FIG_1

FIG_2

# FIG_3

55

F3

12

F4

13

51

# FIG_4

58

55

# FIG_5

10

12

F5

58

56

55

59

36

57

38